# EUROPEAN PATENT APPLICATION

(11) **EP 4 278 981 A1**
(43) Date of publication of application: **22.11.2023**
(21) Application number: 22290032.6
(22) Date of filing: 16.05.2022
(51) Int. Cl.: A61B 8/08, A61B 8/00

(54) **USER GUIDANCE IN ULTRASOUND IMAGING**

(71) Applicant: Koninklijke Philips N.V., 5656 AG Eindhoven (NL)
(72) Inventor: Huiberts, Johannes Nicolaas, 5656 AE Eindhoven (NL); Allain, Pascal, 5656 AE Eindhoven (NL); Sutton, Jonathan Thomas, 5656 AE Eindhoven (NL); Notten, Marc Godfriedus Marie, 5656 AE Eindhoven (NL)
(74) Representative: Philips Intellectual Property & Standards

(57) **Abstract**

The invention provides an apparatus for providing guidance to a user of an ultrasound acquisition system to acquire standardized images of a target anatomical structure of a patient. A processing unit is configured to toggle a user interface between two modes: a guidance mode in which only guidance information is presented on the user interface, and an imaging mode in which a received ultrasound image is presented on the user interface. The processing unit toggles the user interface between these two modes based on a spatial relationship between a current probe position and a target view. When the ultrasound acquisition system is within a threshold distance of the target view then the processing unit toggles the user interface to display the imaging mode, otherwise the guidance mode is displayed.

## Description

### FIELD OF THE INVENTION

This invention relates generally to the field of ultrasound imaging, and in particular to the field of providing guidance information to a user of an ultrasound acquisition system.

### BACKGROUND OF THE INVENTION

Ultrasound imaging is frequently used to obtain images of internal anatomical structures of a patient. Ultrasound systems typically comprise an ultrasound transducer probe that includes a transducer array coupled to a probe housing. The transducer array is activated to vibrate at ultrasonic frequencies to transmit ultrasonic energy into the patient's anatomy, and then receive ultrasonic echoes reflected or backscattered by the patient's anatomy to create an image. These transducers may be used to successively transmit and receive several ultrasonic pressure waves through the various tissues of the body. The various ultrasonic responses may be further processed by an ultrasonic imaging system to display the various structures and tissues of the body.

A sonographer may desire to obtain an ultrasound image representative of a particular view or imaging plane of an organ in order to evaluate the condition of the organ and/or make measurements of the organ. For example, particular acoustic imaging windows of a heart that a sonographer may desire to obtain include "apical," "subcostal" (subxiphoid), "parasternal," and "suprasternal" windows. Obtaining these views involves positioning an ultrasound probe at a specific region of the patient's body and orienting the probe to obtain an image at a desired view. The movements used by the sonographer to position and orient the probe may be complex and may involve several degrees of freedom in three-dimensional space. Accordingly, it can be challenging for inexperienced sonographers to achieve the desired view.

It is important that the image produced by the ultrasound imaging system is as clear and accurate as possible so as to give the user a realistic interpretation of the subject being scanned. This is especially the case when the subject in question is a patient undergoing a medical ultrasound scan. In this situation, the ability of a clinician to make an accurate diagnosis is dependent on the quality of the image produced by the ultrasound imaging system, which in turn is dependent on the ability of a clinician to correctly position the probe in order to acquire a target view of an anatomical structure of interest.

A growing area of interest is the use of ultrasound for whole body, symptom-based exams, in which ultrasound images may be acquired anywhere on the body for fast triaging based on specific symptoms or concerns. Most of these exams are conducted by novice ultrasound operators. Traditionally, ultrasound image views are acquired 'freehand' by experienced users. The probe can be moved without any constraints on position or orientation, and all acquisition settings are configurable. The user is left to decide how and when to acquire images.

However, novice users often do not have sufficient experience in data acquisition and data interpretation to perform complex imaging.

Use of AI algorithms for providing probe guidance and automated image interpretation are already known. In particular, algorithms are known which can interpret acquired ultrasound images and in real-time and generate probe guidance information to be presented to the user, often alongside the acquired images. However, in known systems, the informational content of this guidance is often not sufficient to enable a novice user to perform an imaging procedure. In particular, known forms of guidance can be confusing and overwhelming, especially for inexperienced users in situations where the ultrasound acquisition system, i.e. an ultrasound probe, is starting far from the target anatomical structure they wish to image. The greater affordability of 3D ultrasound probes has made 3D acquisition available to new users, which can make navigation easier due to the volumetric information which is available. However, guidance remains a challenge for inexperienced users.

Another challenge in the field of ultrasound imaging is facilitating more reproducible results. In the present state of the art, quantitative imaging with ultrasound is very difficult because images cannot be relied upon to capture a consistent view. This is true not only for images acquired for different users, but also for a same user navigating to the same view at different instances.

Improved means for providing guidance to an ultrasound user able to address one or more of the above problems would therefore be of value.

### SUMMARY OF THE INVENTION

The invention is defined by the claims.

According to examples in accordance with an aspect of the invention, there is provided an apparatus for providing guidance to a user of an ultrasound acquisition system to acquire a target view of a target anatomical structure of a patient. The apparatus comprises a receiving unit adapted to receive an ultrasound image acquired by the user with an ultrasound acquisition system. The apparatus further comprises a processing unit comprising: a view analyzer module adapted to determine a spatial relationship between the received ultrasound image and a target view, and compute at least one distance metric for the acquired ultrasound image based on said spatial relationship, and a guidance module adapted to generate guidance information based on said spatial relationship, indicative of a change in position and/or orientation of the ultrasound acquisition system for the acquisition of another ultrasound image. The apparatus further comprises an input/output adapted to operably couple the processing unit with a user interface.

The processing unit is adapted in use to selectively toggle the user interface, between: a guidance mode in which guidance information generated by the guidance module is provided on the user interface without the received ultrasound image; and an imaging mode in which the received ultrasound image is provided on the user interface.

The processing unit is adapted to set the user interface to the guidance mode if the at least one distance metric exceeds a predefined threshold, and set the user interface to imaging mode otherwise.

Thus, embodiments provide a guidance scheme for navigating an ultrasound imaging unit toward a target view in which actual ultrasound image information is only presented on-screen once the imaging unit is within a defined spatial zone or window of the target view plane. This improves navigation and reduces human error by constraining the information provided to users of the ultrasound probe while they are still a significant distance away from the target view location. During this phase of navigation, ultrasound image data is of poor quality, providing fuzzy and unclear images which are potentially confusing to the user. Thus, for a novice user, navigation is greatly improved, and human error reduced, by suppressing this image information during the initial probe-navigation phase of the procedure, allowing the user to focus on following the dedicated guidance information which is more intuitive. In other words, navigation is improved by suppressing information which is, in effect, irrelevant or unhelpful to the navigation process due to its poor quality. Such irrelevant or unhelpful information, if not suppressed, is likely to interfere with accurate navigation. Once the user has moved the probe into a threshold proximity of the target view, the image suppression ceases, and the user is free to use the image data to refine the probe placement. At this point, the image information becomes relevant and helpful to the task of positioning the probe.

Existing solutions to probe navigation maintain full navigational freedom for the ultrasound user during the probe navigation phase. The user is required to manipulate the probe based on complex and diffuse imaging information. Non-expert users or new users do not understand this information because it is only indirectly relevant to the process of navigating the probe. Even for expert users, this diffuse imaging information is not directly helpful in navigating the probe, and complicates navigation.

In some embodiments, the guidance information may be output to the user interface in the imaging mode in addition to the received ultrasound image. This allows users to continue to further improve the probe's position so that it may more closely match the target view even when the distance metrics of the ultrasound acquisition system to the target view are all within their predefined thresholds. This also allows more experienced ultrasound users to receive guidance whilst still having access to the images they are acquiring.

In some embodiments, the processing unit may be communicable with a memory storing a database of standardized image views of a plurality of different anatomical structures, and wherein the target view is a view of the target anatomical structure selected from the database. This allows users to select a specific target view of the anatomical structure of which they are interested in acquiring images, the view being one of a set of clinically relevant standardized views.

The processing unit may be communicable with a memory storing a database of previously acquired ultrasound images of the target anatomical structure, and wherein the target view is determined as a view represented by a selected one of the images.

The previously acquired images may be historical images of the target anatomical structure of the same patient. This facilitates clearer comparison between images of a same structure of a patient over time. In particular, the historical image and the image being currently acquired will exactly match in terms of the view represented, which enables changes in clinically relevant information to be more easily detected. For instance, the progression of a disease will be easier to analyze using two images which share the same view.

The previously acquired images may also be historical images of the target anatomical structure of a different patient. This allows users to exactly reproduce a view of an ultrasound image frame of a target anatomical structure of a different patient, facilitating clearer comparison between the two images which may yield clinically relevant information. For instance, comparison of a healthy anatomical structure with an unhealthy anatomical structure will be made easier by the two images sharing the same view.

Each of the images stored in the database may be associated with a set of acquisition settings of the ultrasound acquisition system, and in the imaging mode, the processing unit may be adapted to communicate to the ultrasound acquisition system the corresponding set of ultrasound acquisition settings associated with the selected image. This allows acquired ultrasound images to more reliably match the target view, further increasing the reproducibility of ultrasound images.

In the imaging mode, the processing unit may be further adapted to output electronic steering directions to the ultrasound acquisition system to steer a formed ultrasound beam so that an acquired image matches the target view. This allows acquired ultrasound images to more closely match the target view, further increasing the reproducibility of ultrasound images.

The guidance information may comprise guidance for three degrees of rotational freedom and three degrees of translational freedom. This provides the user clear guidance on probe positioning in all three dimensions in order to arrive at the target view, making the navigation guidance more intuitive and comprehensive. This therefore facilitates the subsequently acquired images to more closely match the target view.

During imaging mode, the processing unit may be adapted to record selected image frames in a memory to compile an examination imaging dataset for the patient, and wherein the processing unit is adapted to only store image frames in the dataset when they match the target view. This again improves reproducibility of acquired images, by constraining image acquisition to only those image frames which match the target view, and thus reducing human error.

The processing unit may be configured to guide the user to acquire a sequence of target views. This may be relevant for instance in the context of performing a symptom examination, in which multiple anatomical structures are of interest in order to investigate the cause of a set of symptoms.

Another aspect of the invention also provides a system for providing guidance to a user of an ultrasound acquisition system to acquire a target view of an anatomical structure, the system comprising the apparatus as described above, or in accordance with any other example or embodiment in this disclosure, and a user interface operably coupled to the input/output of the processing unit.

This system may further comprise an ultrasound acquisition system operably coupled to the receiving unit. This allows the apparatus to communicate directly with an ultrasound acquisition system, facilitating the communication of information such as the instructions for electronic steering and ultrasound acquisition settings to the ultrasound acquisition system.

A further aspect of the invention also provides a computer-implemented method for providing guidance to a user of an ultrasound acquisition system to acquire a target view of a target anatomical structure of a patient, the method comprising receiving an ultrasound image acquired by the user with an ultrasound acquisition system; determining a spatial relationship between the received ultrasound image and a target view, and computing at least one distance metric for the acquired ultrasound image based on said spatial relationship; generating guidance information based on said spatial relationship, indicative of a change in position and/or orientation of the ultrasound acquisition system for the acquisition of another ultrasound image; and toggling a user interface between:
a guidance mode in which guidance information is provided on the user interface without the received ultrasound image; and
an imaging mode in which the received ultrasound image is provided on the user interface;
wherein the method further comprises setting the user interface to the guidance mode if the at least one distance metric exceeds a predefined threshold, and setting the user interface to imaging mode otherwise.

Yet a further aspect of the invention also provides a computer program product comprising code means configured, when executed on a processor, to cause the processor to perform the above computer-implemented method.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a schematic diagram of an apparatus for providing guidance to a user of an ultrasound acquisition system, according to embodiments of the present disclosure;
Fig. 2(a) shows an example of the user interface in guidance mode, according to embodiments of the present disclosure;
Fig. 2(b) shows a further example of the user interface in guidance mode, according to embodiments of the present disclosure;
Fig. 2(c) shows an example of the user interface in imaging mode, according to embodiments of the present disclosure;
Fig. 3 shows an example of a proximal zone in two spatial dimensions of the ultrasound acquisition system, according to embodiments of the present disclosure;
Fig. 4 shows an example of an angular dimension of a proximal zone, according to embodiments of the present disclosure;
Fig. 5 shows a schematic diagram of an apparatus providing guidance to a user of an ultrasound acquisition in communication with a memory, according to embodiments of the present disclosure; and
Fig. 6 shows a flow diagram of a method for providing guidance to a user of an ultrasound acquisition system.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an apparatus for facilitating improved ultrasound probe navigation guidance for acquiring ultrasound images. A navigation scheme is implemented which comprises two navigation phases: one employed while the ultrasound probe is remote from a target image view, and another employed once the probe has been successfully navigated to within a defined field of proximity of the target view. The field of proximity can be defined in terms of translational proximity and optionally also orientational proximity of the probe's current imaging view and the target imaging view. By defining these two phases, the character and content of information provided to a user in each one can be tailored. During the more remote phase, information may be more restrictive, for instance simply giving guidance on moving the probe toward the proximal zone. Once in the proximal zone, the information may additionally include the ultrasound image data acquired by the imaging system, so that the user can adjust the imaging view with greater precision. By suppressing display of the image data during the initial navigation phase, this reduces scope for human error and more narrowly constrains the freedom of adjustment of the user, which ultimately leads to improved navigation to the target imaging view.

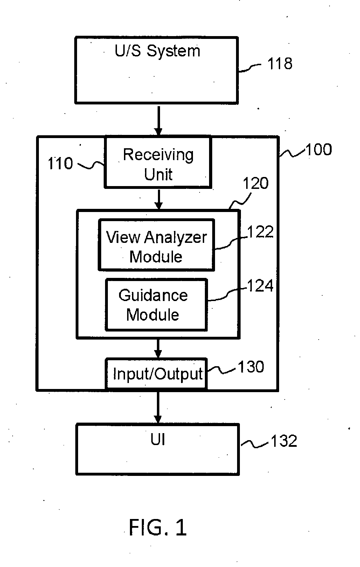

Fig. 1 shows an apparatus 100 in accordance with one or more embodiments for providing guidance to a user of an ultrasound acquisition system ("U/S System") to acquire a target view of a target anatomical structure of a patient. It is noted that the apparatus 100 may be a separate unit from the ultrasound acquisition system 118, as shown in Fig. 1, or may be integrated as a component of the ultrasound acquisition system.

The apparatus 100 comprises a receiving unit 110 adapted to receive an ultrasound image acquired by the user with an ultrasound acquisition system 118. For example, the ultrasound acquisition system may include a probe, the position and orientation of which are freely adjustable by the user by hand. The receiving unit may in some cases receive a series of ultrasound images, e.g. a stream of ultrasound images. It may process each received image in turn.

The apparatus 100 further comprises a processing unit 120, which itself further comprises a view analyzer module 122 and a guidance module 124. It is noted that these may be software modules or hardware modules. Furthermore, although they are shown as separate elements in Fig. 1, they may simply represent software functions or routines, and both may in practice be implemented by a same processing element and/or set of program instructions.

The view analyzer module 122 is adapted to determine a spatial relationship between the received ultrasound image and a target view, and to compute at least one distance metric for the acquired ultrasound image based on said spatial relationship. This distance metric may include a spatial distance part, for instance determined in three dimensions and indicative of a translational distance from the current probe position and a probe position necessary for acquiring the target view. The distance metric may include an orientation component, indicative of a difference between a current probe orientation and an orientation necessary for acquiring the target view. In some embodiments, the view analyzer module 122 may determine a position and orientation of the ultrasound acquisition system (e.g. the probe of the ultrasound acquisition system) based on the received ultrasound image. The position may be defined relative to a reference frame of the anatomy being imaged.

In some embodiments, the target view may be represented in terms of a target position and orientation of the ultrasound acquisition system for acquiring the target view. This may be represented by positional information that includes values associated with one or more physical dimensions or geometric parameters, such as position coordinates for the probe (e.g. x-y-z coordinates, spherical coordinates, cylindrical coordinates), and angular information for the probe (e.g., fanning, rotation, rocking).

The guidance module 124 is adapted to generate guidance information based on said spatial relationship, indicative of a change in position and/or orientation of the ultrasound acquisition system 118 for the acquisition of another ultrasound image closer to the target view.

In some embodiments, the guidance information is for provoking movement by the user of the ultrasound acquisition system that comprises one or more adjustments of the ultrasound transducer unit, for instance including one or more of a lateral sliding movement, a sweeping movement, a rocking movement, a fanning movement, a rotational movement, a compression movement, or a decompression movement.

In the case of 3D ultrasound acquisitions, the acquired volumetric information may be used to help further improve the guidance information and/or electronically steer the probe without the user having to physically re-orient the probe. This is possible because the view presented to the user of a 3D ultrasound acquisition system will often be a 2D image, and so a 2D plane can be selected to present to the user from the 3D information the probe is acquiring.

The apparatus further comprises an input/output 130 adapted to operably couple the processing unit 120 with a user interface 132. The input/output 130 can output guidance information and/or imaging information to the user interface 132.

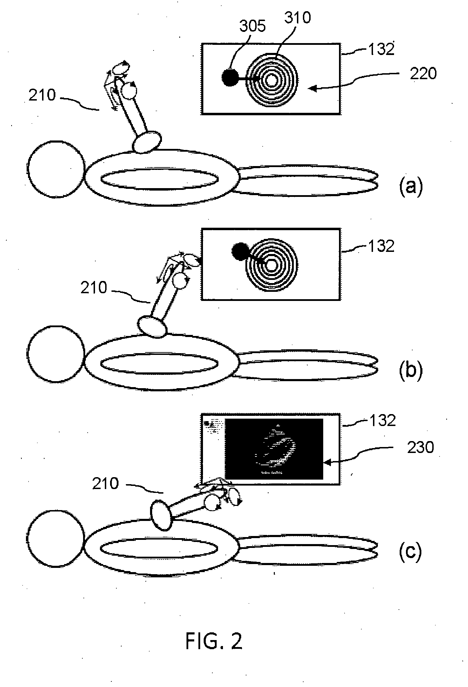

Fig. 2 illustrates use of a system in accordance with one or more embodiments. Figs. 2(a)-(c) show an output to a user interface 132 at different stages throughout probe navigation and imaging during an ultrasound examination of a patient. An example ultrasound imaging probe 210 is illustrated applied to a patient.

Fig. 2(a) illustrates a'start of an examination procedure, in which a user first applies the probe 210 on the body. The apparatus 100 is configured with a defined target view which is to be acquired of a particular target anatomical structure. This may be configured manually in advance by the user, or may be set automatically, for example in accordance with a pre-defined imaging workflow. Options in this regard will be described in greater detail later.

In Fig. 2(a), the probe's 210 starting position is relatively far away from the required position for acquiring the target view. The receiving unit 110 receives an ultrasound image obtained using echo data acquired with the probe at the starting position, and processes the image to determine a distance metric for the acquired ultrasound image indicative of a distance between the current ultrasound image and the target view. For example, the processing unit 120. determines whether the probe is within a threshold distance of the required position for the target view. For example, the processing unit may determine whether the probe is within a defined threshold proximity zone or window or field relative to the required target view, where this may be defined in terms of translational proximity and optionally also orientational proximity of the probe.

In Fig. 2(a), the probe 210 is well outside of the field of proximity from the target view, and so the processing unit 120 determines to keep the apparatus 100 in guidance mode, or to switch the apparatus to guidance mode if the apparatus was previously not in guidance mode.

Fig. 2(a) shows the corresponding output to the user interface 132 during guidance mode. The user interface 205 displays guidance information 220 indicative of a change in position and/or orientation of the ultrasound acquisition system 118 for the acquisition of another ultrasound image. The indicated change is for bringing the probe 210 closer to the position necessary for acquiring the target view. It is for taking the probe closer to the target field of proximity. In some examples, as illustrated in Fig. 2(a), the guidance information may include a visual representation of a relative position of the probe 305 and the proximal zone 310 to convey the positional displacement of the probe from the target view. In guidance mode, guidance information 220 generated by the guidance module 124 is output to the user interface 132 without the received ultrasound image 230. Thus the image information is suppressed.

By suppressing the image information, user error is reduced and navigation accuracy improved. By way of example, this may be especially useful for ultrasound acquisition systems employing 1D probes, where understanding the images on the screen, especially during the navigation process, requires a lot of experience and skill. The imaging information, when the probe is far away from the target view, is confusing and only indirectly relevant to navigation. However, the concept is advantageous for all image acquisition types, including systems using a 2D probe.

In Fig. 2(b), the user has followed the guidance information 220 and moved the probe 210 closer to the target view location. The probe is still outside of the threshold distance from the target view, i.e. outside of the proximal zone, and thus the system remains in guidance mode. Accordingly, only guidance information is shown on the user interface display 132.

With regards to the guidance information, in some embodiments, the guidance information 220 comprises probe movement guidance for one or more degrees of rotational freedom of the probe 210. Preferably, the guidance is for three degrees of rotational freedom of the probe. In some embodiments the guidance information includes movement guidance for three degrees of translational freedom of the probe. This allows the user to receive guidance on how to position the probe in all dimensions, allowing the acquired images to more closely match the target view.

Figs. 2(a) and 2(b) show an example of a simplified 2D picture on the user interface 132 consisting of rings, , wherein this picture is an example of the guidance information 220. The center of the rings is the position corresponding to the target view. The guidance information can include guidance for adjustment of the probe position for example in three degrees of freedom for rotation and three degrees of freedom for translation.

The guidance information may in some examples include a generated visual element for display on a screen which represents, in a single element, guidance information for a plurality of degrees of freedom of the probe, preferable all six degrees of freedom. For example, this may be a generated image which represents the movement guidance for the plurality of degrees of freedom. The guidance information may include static visual indicia, and/or may include moving visual indicia, for example video imagery.

In some embodiments, the guidance information 220 may comprise a rendered visual representation of a virtual landscape or environment, and wherein the movement of the probe 210 is visually rendered as a movement through the virtual landscape or environment, and wherein the user is guided through a virtual trajectory in the landscape/environment, and wherein said virtual trajectory corresponds to a real trajectory of the probe toward the target view location.

Fig. 2 show the user interface 132 in the form of a display unit having a 2D display screen. In some embodiments, the user interface 132 may include a headset or smart glasses for generating guidance information 220 in the form of virtual reality visual information and/or augmented reality visual information.

Fig. 2(c) shows the output to the user interface 132 once the user has moved the probe 210 to within the threshold distance from the target view, i.e. within the proximal zone 310. At this point, the processing unit 120 toggles the apparatus 100 to imaging mode. As illustrated, in imaging mode, the processing unit controls the user interface to display an ultrasound image 230 received from the ultrasound imaging apparatus 118. Thus, in imaging mode, the image information is no longer suppressed and the user can utilize the image information to refine the positioning of the probe.

Optionally, the guidance information 220 may continue to be generated and displayed on the user interface display 132 in the imaging mode, to assist the user in refining the placement of the probe 210. Thus, in some embodiments, there is an option for the guidance information 220 to be output to the user interface 205 in the imaging mode in addition to the received ultrasound image 230. This allows users to continue to further improve the probe's position to more closely match the target view even when the distance metrics are all within their predefined thresholds (e.g. their proximal zones). This also allows more experienced ultrasound users to receive guidance while still having access to the images being acquired. In some examples, whether or not the guidance information is presented to the user during the imaging mode may be determined by the specific target organ or target view to be acquired. An optimization algorithm may be implemented that could adapt the information presented to the user to suit the exact guidance needed for a given target view.

Thus, it can be seen that the processing unit 120 is adapted in use to selectively toggle the user interface 132 between a guidance mode displaying guidance information 220, and an imaging mode displaying a received ultrasound image 230. The processing unit toggles to image mode once the probe 210 moves into sufficient proximity of the target view.

Actual image information 230 is only be shown to the user when the images acquired are within a limited window or proximal zone from the desired anatomy view. In other words, the user interface 132 will only provide ultrasound images to the user when the ultrasound probe 210 is sufficiently close to a position and orientation that will acquire images that match the target view of the target anatomical structure. This simplification reduces margins for human error by withholding confusing image information from the user. An aim is to limit options for novice ultrasound users by minimizing their exposure to complex "fuzzy" image information during their navigation to a target view and instead translating this information into a more intuitive guidance mode in which only guidance information 220 is output to the user interface 132. This ensures there is less human error in navigating to target ultrasound views, and over time the system could learn from previous acquisitions to improve its translations of complex image information to intuitive guidance information.

In practice, the receiving unit 110 may receive a stream of images 230 in real time from the ultrasound acquisition system 118, and may process each in turn with the view analyzer module 122, and re-assess for each image, based on the output of the view analyzer module, which mode the apparatus should be configured in: guidance mode or imaging mode. The image is then either suppressed from display on the user interface in preference of the guidance information 220 in the case of guidance mode, or is displayed on the user interface 132 (optionally in combination with guidance information) in the case of imaging mode. In some embodiments, the guidance module may generate guidance information for every image in both modes. In other embodiments, the guidance module may generate guidance information only in guidance mode, for example responsive to a guidance mode control signal from the view analyzer module or another module of the processing unit.

During imaging mode, the processing unit 120 may be adapted to record selected image frames in a memory to compile an examination imaging dataset for the patient. In some advantageous embodiments, the processing unit may be adapted to only store image frames in the dataset when they match the target view. In other words, capturing ultrasound images is constrained so that only image frames which match the target view can be captured. In some examples, image capture and storage may be controlled automatically by the processing unit, e.g. via a control signal sent along a communication link with the ultrasound acquisition system dependent upon the present image view matching the target image view. In other examples, the user may trigger image capture and storage, but wherein this is constrained so that a user is only able to capture and store images when the target image view is matched.

This again improves reproducibility of acquired images, by constraining image acquisition to only those image frames which match the target view, and thus reducing human error.

In some embodiments, the processing unit 120 may be adapted to compute a quantitative metric or score indicative of an image similarity/dissimilarity between a newly captured image and the target image view. This provides an image quality metric. The captured image may be labelled or tagged with the metric or score when it is saved in the examination imaging dataset for the patient.

With regards to the generating of the guidance information 220, there exist known solutions for achieving this.

For example, there exist known AI tools for generating ultrasound probe navigation guidance information 220. At least some of these tools are adapted to classify and annotate images acquired from a region outside of the threshold distance from the target image view, and to translate the image information into user interface navigation information to support users in reaching the target proximal zone.

One such example is described in US 2021/000446, which details the use of at least one convolutional neural network as part of a predictive network which is applied to an acquired ultrasound image to obtain a motion control configuration for repositioning an imaging device from a first imaging position to a second imaging position. The motion control configuration is used to generate instructions to be displayed to the user which may prompt them to operate a control component in communication with the imaging device such that the imaging device is repositioned to the second imaging position closer to a desired location for capturing a target image view. This tool could be used in the current application to help generate navigation guidance information 220.

Although in the above-described example, the ultrasound acquisition system 118 is described as comprising a probe 210, and wherein the guidance information 220 is for guiding movement of the probe, the inventive concept is applicable for any type of ultrasound transducer unit. For example, instead of a probe, the ultrasound transducer unit could take the form of a patch having one or more ultrasound transducers integrated therein. An ultrasound patch is useful for longer term ultrasound monitoring functions. The guidance information is in this case for guiding placement of the patch. Patches typically have capacity to scan the anatomy, with 3D or 2D functionality, and so, during positioning of the patch, if the patch is too far from the target view, guidance information may be used to improve placement.

As discussed above, the processing unit 120 is adapted to determine a distance metric for each acquired ultrasound image based on a determined spatial relationship between the received ultrasound and the target view. The processing unit then uses the distance metric, with reference to a threshold, to determine whether the apparatus should be toggled to guidance mode or to imaging mode. Options for deriving a distance metric and for selecting the mode based on a distance metric threshold will now be briefly discussed.

One preferred approach according to one or more embodiments, is to define the distance metric threshold in terms of a target field or proximal zone relative to the target view. The field or proximal zone can be defined in terms of translational proximity and optionally also orientational/rotational proximity of the current imaging view of the ultrasound transducer unit and the target imaging view. At minimum it defines a threshold translational distance of the ultrasound transducer unit from the position associated with the target view.

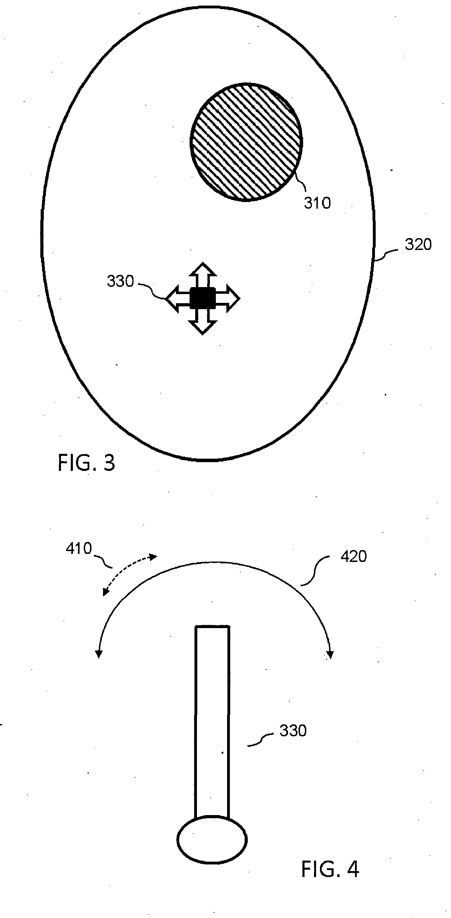

Fig. 3 and Fig. 4 schematically illustrate examples of predefined thresholds for distance metrics in the form of target proximal zones.

Fig. 3 schematically illustrates an example of a target lateral proximal zone 310 on the lateral surface of a body 320 of the patient. The boundaries of the lateral proximal zone are defined by a threshold lateral distance between a current position of the ultrasound transducer unit and a target position required for acquiring the target view. The target position is for example at a center of the proximal zone 310.

If the ultrasound acquisition system 330 is within this proximal zone 310 the processing unit 120 may set the user interface 132 to imaging mode, and otherwise the user interface may be set to guidance mode. The target lateral proximal zone 310 represents the zone, outside of which, the apparatus 100 is kept in guidance mode.

Fig. 4 provides an example of an optional target rotation proximal zone 410 on an axis of rotation 420. Optionally, the system may be kept in guidance mode until the probe is within a certain range or field of the target orientation of the probe for acquiring the target view. If the ultrasound acquisition system 330 is within this proximal zone 410 the processing unit 120 may set the user interface 132 to imaging mode, and otherwise the user interface may be set to guidance mode. The target rotation proximal zone 410 represents the zone in orientation space outside of which, the apparatus 100 is kept in guidance mode.

The processing unit 120 may be configured to set the user interface 132 to imaging mode only if the ultrasound acquisition system 330 is within at least a subset of all translational and rotational proximal zones, and otherwise the user interface may be set to guidance mode.

Proximal zones may be determined by translation and/or rotation deviations between the probe current pose and the probe pose needed to obtain a target view. A co-ordinate system may be defined which includes three rotational and three translational co-ordinates, and wherein the current probe position is defined or represented with reference to the co-ordinate system and wherein the proximal zone is defined or represented with reference to the same co-ordinate system.

The predefined threshold for the distance metric may be personalized for a given user of the apparatus 100. The predefined threshold for the distance metric may be adjusted over time for a given user. For example, as user skill increases, the threshold may be changed so that the proximal zone which defines the toggling to imaging mode is made narrower, i.e. so that the user must navigate closer to the target view before imaging mode is triggered.

With regards to the target view to be acquired, there are different options as to how this may be configured or selected in advance of the guidance and imaging operation.

In some embodiments, the processing unit 120 may be communication with a memory storing a database of pre-defined image views of a plurality of different anatomical structures. In this embodiment, the target view may be selected (automatically or manually) as a view of a given target anatomical structure selected from the database. It may be selected manually by a user in advance of beginning an examination, or may be selected automatically, for example in accordance with a predefined imaging protocol or when a proximity of the ultrasound probe to a certain anatomical structure is registered/detected.

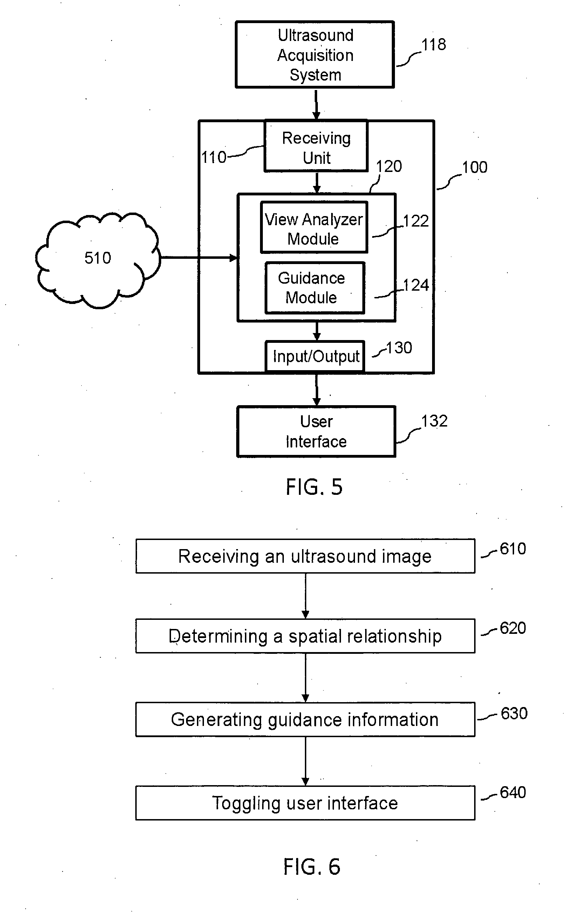

Fig. 5 illustrates an example apparatus in which the processing unit 120 is arranged in communication with a memory 510 storing a database of pre-determined image views. In the illustrated example, the memory is in the form of a cloud-based memory or data-store. The processing unit may be arranged to communicate with the cloud based memory via an internet or network link. However, in other examples, the memory 510 may instead be a local memory. At least a subset of the pre-defined image views may correspond to clinically standardized image views of each of the plurality of different anatomical structures. This means image views which form part of a clinically standard imaging protocol for a given anatomical structure. For example, for imaging the heart, standardized image views include Parasternal Long Axis, Parasternal Short Axis, Apical Four Chamber, and Subcostal Four Chamber, among others.

When standardized anatomy views are used, this may be referred to as a "Standard Acquisition Setting" mode, however it may also be referred to by any other suitable name. The views are defined relative to the position and/or rotation angle of anatomy in the image. This allows users to select a specific target view of the anatomical structure to be imaged, and wherein the view is guaranteed to be a standardized clinically relevant view.

Each predefined image view in the database may be labelled with a name so that it can be identified, and may be associated with a particular pose or position of the probe 210 relative to the relevant anatomical structure, which may include a translational position and an orientation position of the probe.

In some embodiments, the processing unit 120 is communicable with a memory 510 storing a database of previously acquired ultrasound images of the target anatomical structure, and wherein the target view is determined as a view represented by a selected one of the images. Thus in this case, the database does not store a direct representation of image views, but rather stores images which each represent a particular view.

Within this set of embodiments, a number of different options again exist.

In some embodiments, the previously acquired images stored in the database may include historical images of the target anatomical structure of the same patient who is being examined. This allows users to exactly reproduce a view of an ultrasound image of a target anatomical structure of the same patient. In particular, this will enable changes in clinically relevant information to be more easily detected. For instance, the progression of a disease will be easier to analyze using two images which share the same view. In some embodiments, the processing unit 120, in imaging mode, may permit a user to upload a newly acquired image to the patient-specific database of image views, to be later used as a target view in a subsequent examination.

In further embodiments, the previously acquired images stored in the database may include historical images of the target anatomical structure of a different patient than the one being examined. This allows users to exactly reproduce a view of an ultrasound image of a target anatomical structure of a different patient, facilitating clearer comparison between the two images, which may yield clinically relevant information. For instance, comparing a healthy anatomical structure to an unhealthy anatomical structure may be easier when the images are of the same view. This approach is also helpful where the patient has never been examined before, and so no patient-specific historical images exist.

In some embodiments, the database may store a set of previously acquired ultrasound images which were acquired by particular other users, and which are labelled with the name, or another identifier, of the user who acquired them. For example, the images may be images acquired by an expert or key opinion leader. The target view is set as a view represented by a selected one of these expert-acquired images. In this case, the database might be stored on a cloud-based datastore 510 which is accessible to a plurality of different users, potentially at different institutions. The acquisitions stored in the database may include all acquisition information and may potentially include diagnostic outcome information. In this way, ultrasound users may continuously improve their acquisition skills by having access to this information and learning from the prior acquisitions of experts. The standardized views may be labelled in this database as being acquired by a particular user. This approach greatly enhances image acquisition quality and reproducibility, since even new users can acquire image frames which exactly match an imaging view as acquired by an expert user.

Standardized or expert or key opinion leader anatomy views could be delivered as a service. For example, an online portal may be established which permits access to sets of pre-acquired imaging views from certain experts, wherein access to download the imaging views is constrained to authorized users, for example in accordance with a subscription program which must be paid for. Standard anatomy views could also be part of standard protocols in medical facilities, for example a standard protocol which defines a particular sequence of imaging views to be captured. Incidental findings may be minimized by using expert-defined imaging views.

The use of previously acquired images to represent the target views ensures optimal reproducibility for a range of uses.

For example, with 2D probes, 3D information is acquired which can then be used to further improve the guidance information or allow electronic steering of the probe. Once a 3D scan has been acquired by the 2D probe by acquiring information from multiple planes in the volume, electronic steering can then allow the 2D probe to acquire images from a target plane without any physical movement. This information could be used to create a transfer function to an image that is as close as possible to a previously acquired image.

Such reproducibility will optimally support extraction of quantitative data from the acquired image.

In some embodiments, the processing unit is configured to guide the user to acquire a sequence of target views. This imaging of a sequence of target anatomical structures, which may be referred to as a navigation trajectory, may be based on previously acquired images acquired by expert users and/or key opinion leaders. This could be augmented with a dedicated graphical user interface. This dedicated graphical user interface would allow ultrasound users to more easily receive guidance in performing a symptom examination, wherein multiple anatomical structures are of interest, and so the user may be guided to acquire target views of a sequence of anatomical structures.

In some embodiments, each of the images stored in the memory 510 is associated with a set of acquisition settings of the ultrasound acquisition system 118. In the imaging mode, the processing unit 120 may be adapted to communicate to the ultrasound acquisition system the corresponding set of ultrasound acquisition settings associated with the selected image. This allows acquired ultrasound images to more closely match the target view, further increasing the reproducibility of ultrasound images.

In some embodiments, additional sensors may be implemented, such as an inertial measurement unit in the probe, for sensing probe orientation. This allows to even further refine the probe positioning and improve the reproducibility of ultrasound images.

In another embodiment, standardized view settings may be communicated to the ultrasound acquisition system 118 when the probe 210 approaches the target view, or the settings may automatically be set before the acquisition.

In some embodiments, during imaging mode, the processing unit 120 may be adapted to output electronic steering directions to the ultrasound acquisition system 118 to steer a formed ultrasound beam so that an acquired image matches the target view. Typically, this is for 2D probes that have the ability to acquire 3D imaging information. This can ensure increased reproducibility with respect to previously acquired acquisitions being selected as the target view. With 3D sensors, where no manipulation is possible, this feature can be included to ensure increased stability of images.

Another aspect of the invention also provides a system for providing guidance to a user of an ultrasound acquisition system 118 to acquire a target view of an anatomical structure, the system comprising: an apparatus 100 in accordance with any of the examples or embodiments outlined in this disclosure: and a user interface 132 operably coupled to the input/output 130 of the processing unit 120.

This facilitates communication between the apparatus 100 and a user interface 132 which can display the guidance 220 and imaging 230 information to the user.

This system may in particular embodiments further comprise an ultrasound acquisition system 118 operably coupled to the receiving unit 110. This allows the apparatus 100 described above to communicate with an ultrasound acquisition system, facilitating the communication of information such as the instructions for electronic steering and ultrasound acquisition settings to the ultrasound acquisition system.

Fig. 6 outlines steps of a computer-implemented method for providing guidance to a user of an ultrasound acquisition system 118 to acquire a target view of a target anatomical structure of a patient.

In step 610, an ultrasound image acquired by the user with an ultrasound acquisition system 118 is received.

In step 620, a spatial relationship is determined between the received ultrasound image and a target view, and at least one distance metric for the acquired ultrasound image is computed based on said spatial relationship.

In step 630, guidance information 220 is generated based on said spatial relationship, indicative of a change in position and/or orientation of the ultrasound acquisition system for the acquisition of another ultrasound image.

In step 640, the user interface 132 is toggled between: a guidance mode in which guidance information is provided on the user interface without the received ultrasound image; and an imaging mode in which the received ultrasound image 230 is provided on the user interface, wherein the processing unit 120 is adapted to set the user interface to the guidance mode if the at least one distance metric exceeds a predefined threshold, and set the user interface to imaging mode otherwise.

Another aspect of the invention provides a computer program product comprising code means configured, when executed on a processor, to cause the processor to perform the above computer-implemented method.

The various embodiments of the present invention described above provide a number of advantages in the context of ultrasound imaging.

Embodiments described above advantageously constrain the imaging information provided to users of an ultrasound acquisition system, which thereby avoids presenting poor quality and potentially confusing images to the user, allowing the user to focus on following the more intuitive guidance information 220. Furthermore, guiding the user towards acquiring images matching a target view increases the reproducibility of ultrasound images. In that regard, embodiments of the present disclosure advantageously provide an intuitive, probe-centric interface for guiding an ultrasound imaging procedure that involves fewer mental operations or translations for an operator to follow on-screen instructions to move the ultrasound acquisition system.

Embodiments provide a benefit not only for inexperienced users but also provide the benefit of improved reproducibly even for expert users. Embodiments improve navigation accuracy and speed. For example, the use of previously acquired ultrasound images can aid experienced users in carrying out ultrasound exams faster in subsequent examinations, relying on the guidance information 220 to ensure they reach the same target view as a previously acquired image.

The embodiments disclosed herein may facilitate improved telehealth solutions, manipulating probes at a distance or helping inexperienced users to manipulate the probe.

The present invention may also find use in rapid ultrasound whole body exams by novice and irregular users in and outside of the hospital. With developments in the field, reducing the formfactors of ultrasound systems, the number of ultrasound users are expected to increase 10-50 fold in the next 5-10 years. Embodiments of the invention described above employ a processing unit. The processing unit may in general comprise a single processor or a plurality of processors. It may be located in a single containing device, structure or unit, or it may be distributed between a plurality of different devices, structures or units. Reference therefore to the processing unit being adapted or configured to perform a particular step or task may correspond to that step or task being performed by any one or more of a plurality of processing components, either alone or in combination. The skilled person will understand how such a distributed processing arrangement can be implemented. The processing unit may include a communication module or input/output for receiving data and outputting data to further components.

The one or more processors of the processing unit can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner/

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. An apparatus for providing guidance to a user of an ultrasound acquisition system to acquire a target view of a target anatomical structure of a patient, the apparatus comprising:
a receiving unit (110) adapted to receive an ultrasound image acquired by the user with an ultrasound acquisition system;
a processing unit (120) comprising:
a view analyzer module (122) adapted to determine a spatial relationship between the received ultrasound image and a target view, and compute at least one distance metric for the acquired ultrasound image based on said spatial relationship, and
a guidance module (124) adapted to generate guidance information based on said spatial relationship, indicative of a change in position and/or orientation of the ultrasound acquisition system for the acquisition of another ultrasound image; and
an input/output (130) adapted to operably couple the processing unit with a user interface,
wherein the processing unit is adapted in use to selectively toggle the user interface, between:
a guidance mode in which guidance information (220) generated by the guidance module is provided on the user interface without the received ultrasound image; and
an imaging mode in which the received ultrasound image (230) is provided on the user interface; and
wherein the processing unit is adapted to set the user interface to the guidance mode if the at least one distance metric exceeds a predefined threshold, and set the user interface to imaging mode otherwise.

2. The apparatus of claim 1, wherein the guidance information is output to the user interface in the imaging mode in addition to the received ultrasound image.

3. The apparatus of claims 1 or 2, wherein the processing unit is communicable with a memory (510) storing a database of standardized image views of a plurality of different anatomical structures, and wherein the target view is a view of the target anatomical structure selected from the database.

4. The apparatus of claims 1 or 2, wherein the processing unit is communicable with a memory storing a database of previously acquired ultrasound images of the target anatomical structure, and wherein the target view is determined as a view represented by a selected one of the images.

5. The apparatus of claim 4, wherein the previously acquired images are historical images of the target anatomical structure of the same patient.

6. The apparatus of claim 4, wherein the previously acquired images are historical images of the target anatomical structure of a different patient.

7. The apparatus of claim 4, wherein,
each of the images stored in the database is associated with a set of acquisition settings of the ultrasound acquisition system; and
in the imaging mode, the processing unit is adapted to communicate to the ultrasound acquisition system the corresponding set of ultrasound acquisition settings associated with the selected image.

8. The apparatus of any of claims 1-7, wherein, in the imaging mode, the processing unit is further adapted to output electronic steering directions to the ultrasound acquisition system to steer a formed ultrasound beam so that an acquired image matches the target view.

9. The apparatus of any of claims 1-8, wherein the guidance information comprises guidance for three degrees of rotational freedom and three degrees of translational freedom.

10. The apparatus of any of claims 1-9, wherein during imaging mode, the processing unit is adapted to record selected image frames in a memory to compile an examination imaging dataset for the patient, and wherein the processing unit is adapted to only store image frames in the dataset when they match the target view.

11. The apparatus of any of claims 1-10, wherein the processing unit is configured to guide the user to acquire a sequence of target views.

12. A system for providing guidance to a user of an ultrasound acquisition system to acquire a target view of an anatomical structure, the system comprising:
the apparatus of any of claims 1-11; and
a user interface operably coupled to the input/output of the processing unit.

13. The system of claim 12, further comprising an ultrasound acquisition system operably coupled to the receiving unit.

14. A computer-implemented method for providing guidance to a user of an ultrasound acquisition system to acquire a target view of a target anatomical structure of a patient, the method comprising:
receiving an ultrasound image acquired by the user with an ultrasound acquisition system;
determining a spatial relationship between the received ultrasound image and a target view, and computing at least one distance metric for the acquired ultrasound image based on said spatial relationship;
generating guidance information based on said spatial relationship, indicative of a change in position and/or orientation of the ultrasound acquisition system for the acquisition of another ultrasound image; and
toggling a user interface between:
a guidance mode in which guidance information is provided on the user interface without the received ultrasound image; and
an imaging mode in which the received ultrasound image is provided on the user interface;
wherein the method further comprises setting the user interface to the guidance mode if the at least one distance metric exceeds a predefined threshold, and setting the user interface to imaging mode otherwise.

15. A computer program product comprising code means configured, when executed on a processor, to cause the processor to perform the computer-implemented method of claim 14.
